# EUROPEAN PATENT APPLICATION

(11) **EP 3 536 381 A1**
(43) Date of publication of application: **11.09.2019**
(21) Application number: 19166049.7
(22) Date of filing: 21.10.2010
(51) Int. Cl.: A61P 19/10, A61K 31/075, A61K 31/191, A61K 31/194, A61K 31/198, A61K 35/64, A61K 35/63, A23L 33/10, A23L 33/16

(54) **BIOLOGICALLY ACTIVE FOOD SUPPLEMENT FOR USE IN PREVENTING OSTEOPOROSIS**

(30) Priority: 30.11.2009 RU 2009144461
(62) Divisional of application: 10833641.3
(71) Applicant: Obshestvo S Ogranichennoj Otvetstvennostju "PARAFARM", Penza 440026 (RU)
(72) Inventor: ELISTRATOV, Dmitriy Gennadjevich, 440060 Penza (RU)
(74) Representative: Engel, Christoph Klaus

(57) **Abstract**

The invention relates to biologically active food supplements and is intended for preventive action against the states associated with osteoporosis. The technical effect consists in providing a biologically active food supplement which sustains a normal testosterone level in the body, ensures an efficient assimilation of calcium by the body and retention thereof in the bony tissue for a prolonged time enabling thereby the prevention of osteoporosis.

## Description

The present invention relates to biologically active food supplements and is intended for preventive action against the states associated with osteoporosis.

The problem of osteoporosis (reduction in the amount of calcium in the bone structures) is a common knowledge. In order to address this problem, calcium preparations are used based on calcium carbonate, calcium citrate, calcium gluconate, calcium aspartate, calcium ascorbate, calcium aminochelate, calcium fumarate, calcium succinate, calcium phosphate, calcium limonene oxide and other compounds. It is known that calcium preparations are better assimilated together with vitamin D. Therefore, multiple drugs are known, for example, calcium gluconate tablets (registration number R No. 000140/02-2001 MZ RF), Calcium D3 Nycomed tablets (registration number P No. 013478/01-2001) and the like.

The prior art preparations have the following drawbacks:
1) Unfortunately, all known preparations are directed only to increasing the ingress of calcium into the body rather that eliminating the reasons of depletion of calcium from the bones.
2) When a calcium preparation is administered, calcium may enter not only the bones but also other organs and systems, in particular it may calcify concrements in the kidneys leading to a malfunction of kidneys.

In other words, to treat osteoporosis with calcium preparations is as effective as trying to pour water into holed barrel. It is of greater importance the ability of body to assimilate calcium and to use the same as intended rather than the amount of calcium being administered.

On the other hand, it is known that an important role in preserving the density of the mineral bone stock in both young and elderly men is played by androgens, i.e. male sex hormones. A lower bone density and a higher incidence of bone fractures is observed in men suffering from hypogonadism (i.e. reduced level of the male sex hormone, i.e. testosterone). A low testosterone level is one of the major causes of osteoporosis, i.e. lowered mineral bone density in men, and is hence a risk factor of fractures.

An average testosterone level in women is 25 times lower than that in men so that even an insignificant decrease in the testosterone level leads to osteoporosis. This also why, more women than men suffer from osteoporosis.

Male sex hormones or androgens (first of all testosterone) influence the bony tissue both directly and indirectly. Testosterone directly affects the bony tissue and is responsible for the differences in the structure of male and female skeletons. Testosterone indirectly affects the bone formation through estrogens, female sex hormones which are formed from androgens under the action of aromatase. Since testosterone is the primary estrogen in males, a decrease in its secretion also leads to a decrease in the bone density.

Accordingly, men with diagnosed osteoporosis need to undergo hormonal examination including tests for testosterone, estradiol and sex steroid-binding globulin. In turn, all men with a low content of sex hormones need to undergo densitometry with a view to detecting osteoporosis.

Various medicaments are used for the treatment of decreased bone density and osteoporosis, such as calcium preparations, vitamins D and bisphosphonates. However, it has been proved that such medicaments have a minimum efficiency given that the testosterone level is still low, because the proper assimilation of medicaments directed to repairing the bone density is ensured precisely by testosterone. Therefore, the treatment of the bone density disorders has to be comprehensive, directed to both compensating for a deficit (if any) in testosterone and administering of calcium preparations.

However, when putting this theory in practice problems have been faced with the known preparations sustaining the testosterone level in the body (for example, testosterone propionate) since they all require the most stringent health control.

The reason is that testosterone preparations are referred to doping drugs and have a lot of negative effects on the human body. As a result of administering testosterone from the outside, production of its own testosterone in the human body decreases even more. On the other hand, as shown above, the body is in constant need for the ingress of calcium.

Therefore, it is an object of the present invention to provide a biologically active food supplement for ongoing administration, which would compensate for the content of calcium in the body, sustain the testosterone level and also facilitate a better assimilation of calcium and retention thereof in the body.

Said object is achieved by providing a biologically active food supplement for the prevention of osteoporosis, comprising on the weight percent basis:
a calcium compound selected from calcium carbonate, calcium citrate, calcium gluconate, calcium aspartate, calcium phosphate and calcium limonene oxide - 16.67 to 93.75 wt. %;
a drone brood - 6.25 to 83.33 wt. %.

The inventive biologically active food supplement is available in a powdered, tablet or capsular form.

The technical effect consists in providing a biologically active food supplement which sustains a normal testosterone level in the body, ensures an efficient assimilation of calcium by the body and retention thereof in the bony tissue for a prolonged time enabling thereby the prevention of osteoporosis.

The use of the drone brood is explained by the following. The drone brood is a donator of sex entomological hormones: prolactin, estradiol, prohesterone, testosterone which stimulate the reproductive functions in men and women. Rich in hormones and vitamins not being hormone substitutes, the drone brood is effective against the hormonal background disorders, stimulates the central mechanisms for regulating the rate of androgen formation and excludes possible replacement therapy. The studies conducted in the apiculture scientific-research institute (Rybnoye, Ryazan region, Russia) demonstrated the safe use of the drone brood and also proved the gonadotropic effect of the drone brood on the stimulation of central links controlling the testosterone synthesis. (Krivtsov N.I. et al., Theory and Methods of Apitherapy, Moscow, 2007). Therefore, the testosterone level in the body may be sustained using the drone brood.

Although the components forming part of the inventive biologically active food supplement are known in the folk or traditional medicine, their combination is not disclosed in any of the prior art products.

The combined use of said components enables a more efficient assimilation of calcium by the body since calcium not only enters the body but is also retained in the bony tissue thanks to the drone brood. The inventors have found that the drone brood is able to improve the assimilability of calcium by the body and to retain the same in the bony tissue for a long time, i.e. to ensure a prolonged effect of the administration of calcium into the bony tissue. In addition, the administration of calcium together with the drone brood makes it possible to lower the dosage of the calcium compounds for avoiding lithogenesis in the kidneys and other negative effects of the calcium preparations (for example, gastroenteric upsets).

The ratio of the calcium compound and the drone brood varies within a wide range. The range width is determined by the individual parameters: age, gender, state of health and other factors.

It is known that the upper acceptance limit for the administration of calcium is 1000-1500 mg per day. Due to the combined administration of the drone brood and the calcium compound the daily dosage of calcium may be lowered since the bioavailability (assimilability) of calcium is improved and bone resorption (calcium depletion) is prevented.

Based on the foregoing, the maximum consumption of calcium is 600 mg.

The minimum consumption of calcium is determined by the minimum body need for ionized calcium being of 200 mg.

The maximum consumption of the drone brood is determined by the expediency of use and is 1000 mg.

The minimum consumption of the drone brood is 40 mg (see RU 2233666 C1, 10.08.2004) and is determined by the pharmacological activity.

Therefore, the administration range of the components forming part of the inventive biologically active food supplement is as follows:
drone brood: 40 to 1000 mg or 6.25% to 83.33%;
calcium: 200 to 600 mg or 16.67% to 93.75%.

Thus, the present invention regards a biologically active food supplement in the powdered, tabletted or capsulated form intended for the prevention of osteoporosis, comprising on the weight percent basis:
a calcium compound selected from:
   calcium carbonate,
   calcium citrate,
   calcium gluconate,
   calcium aspartate,
   calcium phosphate,
   calcium limonene oxide 16.67 to 93.75;
drone brood 6.25 to 83.33.

Therefore, the combined administration of the drone brood and calcium compound in the supplement makes it possible to reduce significantly the maximum (upper) daily consumption of calcium from 1500 mg to 600 mg thereby reducing the negative side effects of calcium on the human body and widening the group of persons able to use calcium preparations. A reduction in the maximum daily consumption of calcium is necessary because another disease, hypercalcemia quiet often occurs otherwise in cases where calcium preparations are administered in the doses of 1000 to 1500 mg. Thus, of the 1000 patients continuously receiving preparations against osteoporosis, 86 had hypermineralization of the 1 to 3 degree (Strukov V.I., Actual Problems of Osteoporosis, Penza, 2009, Rostra Publishers).

The above-identified quantitative ratio makes it possible to ensure an optimum ingress of calcium for each individual and its most complete assimilation thanks to the inventive amount of the drone brood which provides a certain content of sulfhydryl enzyme groups in the supplement, as well as testosterone, prohesterone, prolactin and estradiol. The drone brood is rich in amino acids. The amino acids in turn act as transporters of calcium to the cell (Strukov V.I., Actual Problems of Osteoporosis, Penza, 2009, Rostra Publishers). In order for calcium to be assimilated, vitamins A, C, E, D and micronutrient elements, such as magnesium, copper, zinc, phosphorus are also required. All of them are present in the drone brood.

As a result, all calcium being administered is completely assimilated by the bony tissue. It has been surprisingly found that the retention of calcium in the bony tissue for a prolonged time is provided by a certain amount of sulfhydryl enzyme groups in the drone brood, vitamins D and E so that the process of its depletion slows down. Besides, a long period of the normal bony tissue density is accounted for by a sufficiently high content of phosphorus in the drone brood which enters the body in the amount specified above thereby facilitating the assimilation of calcium.

Therefore, the problem of not only saturating the bony tissue with calcium but also of retaining the same for a long time therein has been solved for the first time by means of the combined use of the calcium preparations and the drone brood in the proposed ratio. The risk of fractures has been considerably lessened as compared to the prior art preparations since a 5% increase in the mineral bone density leads to a 35% reduction in the risk of fracture, and the additional antiresorptive activity of the inventive preparation reduces the risk of fracture by another 20%.

In order to prepare the inventive biologically active food supplement, a powdered calcium compound is provided, selected from calcium carbonate, calcium citrate, calcium gluconate, calcium aspartate, calcium phosphate, calcium limonene oxide at a concentration of 16.67 wt. % to 93.75 wt. % and is mixed with a drone brood at a concentration of 6.25 wt. % to 83.33 wt. % until a homogeneous mixture is formed. The resulting mixture is then tabletted or capsulated. The biologically active food supplement comprises a homogeneous light beige powder with a humidity of 1 to 3.5%, which is subjected to tabletting or capsulation. A tablet or capsule contains 1 g of mixture.

The drone brood for preparing the biologically active food supplement is used in the form of a lyophilizate. In order to obtain a lyophilizate, the larval drone brood is homogenized using a mixer. The homogenate comprises a thick yellow creamy liquid with a characteristic odor. The homogenate has a content of solid matter not exceeding 22.5%. The lyophilizate is obtained by freezing the homogenate for 2-3 hours at a temperature of minus 35-40°C followed by vacuum sublimation at a residual pressure of 0.1-0.6 mm Hg for 40-48 hours and bringing the temperature to 25-30°C by the end of lyophilization. The lyophilizate comprises a powder whose color varies from beige to yellow, with a high hygroscopicity and residual moisture content of 1-3.5%. In other words, the preparation conditions of lyophilizate are such that the solid matter composition of the drone brood homogenate may be preserved without changes except for the moisture content.

Below are provided the examples of preparing the biologically active food supplement.

### Example 1

17 kg of powdered calcium gluconate was provided and mixed with 83 kg of the drone brood lyophilizate. The mixer was stirred to homogeneity and the resulting product was capsulated.

### Example 2

90 kg of powdered calcium carbonate was provided and mixed with 10 kg of the drone brood lyophilizate. The mixer was stirred to homogeneity and then tabletted.

For prevention of osteoporosis, one capsule of the biologically active food supplement was administered 3 times a day for one month separately from meals. A one-month break in the administration should be provided. The serum calcium value does not reduce during this period. In other words, the calcium content is sustained at a normal level.

The above technical effect is demonstrated by way of the following examples.

### Example 1

Human subject, female, born in 1959. The mineral bone density of T -1.4 was measured in the distal antibrachium by means of DXT-200 densitometric apparatus available from Osteometr. Osteopenia was diagnosed and 1 g of calcium gluconate was administrated 3 times a day. A six-month course of preventive medication was conducted. The reduced bone density of T -1.6 was measured after six moths during the re-examination. That is, the mineral bone density (MBD) became less. After a one-month break in the administration, the serum calcium value was below the normal value.

For reference:
MBD normal value - the values of T criterium with a standard deviation of +1 to -1 from the peak bone mass;
1^{st} degree osteopenia- MBD of -1 to - 1.5;
2^{nd} degree osteopenia - MBD of -1.5 to -2;
3^{rd} degree osteopenia - MBD of -2 to -2,5;
1^{st} degree osteoporosis - MBD of -2.5 and less without fractures;
2^{nd} degree osteoporosis - MBD of -2.5 and less with osteoporotic bone fractures.

The densiometric T scale presents the number of standard deviations up and down an average bone mass value. The T criterium decreases in parallel with a gradual reduction in the bone mass with the increase of years and is used to evaluate the MBD in adults.

### Example 2

The same female human subject with diagnosed osteopenia and the mineral bone density of T- 1.6 was administered one capsule of the biologically active food supplement 3 times a day. A six-month preventive medication course was conducted following the pattern of one-month administration/one-month break. The mineral bone density increased to T -1.2. The serum calcium value normalized. No reduction in the serum calcium value was observed during the re-examination performed three months later.

Therefore, apart from sustaining the normal testosterone level and compensating for the deficit of calcium in the body, the inventive biologically active food supplement also facilitates a more efficient assimilation of calcium and retention thereof in the bony tissue for a long time (i.e. has a prolonged activity) and thereby is an excellent preparation for prevention of osteoporosis.

## Claims

1. A biologically active food supplement for use in preventing osteoporosis comprising
ionized calcium 200 mg to 600 mg
drone brood 40 mg to 1000 mg
being administered from 1 to 3 times a day.

2. The biologically active food supplement for use in preventing osteoporosis according to claim 1, wherein said ionized calcium is provided contained in:
calcium carbonate,
calcium citrate,
calcium gluconate,
calcium aspartate,
calcium phosphate.

3. The biologically active food supplement for use in preventing osteoporosis according to claim 1, wherein drone brood is provided in form of lyophilizate.
